Europäisches Patentamt

**European Patent Office**

**Office européen des brevets**

(11) Publication number: **0 365 756**

**A1**

## EUROPEAN PATENT APPLICATION

(21) Application number: **89112728.4**

(22) Date of filing: **12.07.89**

(51) Int. Cl.5: **C12M 1/24 , B01L 3/00 , C12M 1/04**

The title of the invention has been amended (Guidelines for Examination in the EPO, A-III, 7.3).

(30) Priority: **26.08.88 US 236956**

(43) Date of publication of application:
**02.05.90 Bulletin 90/18**

(84) Designated Contracting States:
**CH DE FR GB LI NL**

(71) Applicant: **LOFSTRAND LABS LIMITED**
**7961 Cessna Avenue**
**Gaithersburg Maryland 20879(US)**

(72) Inventor: **Alexander, Robert**
**1700 Chester Mill Road**
**Silver Spring Maryland 20906(US)**

(74) Representative: **Füchsle, Klaus, Dipl.-Ing. et al**
**Hoffmann . Eitle & Partner Patentanwälte**
**Arabellastrasse 4**
**D-8000 München 81(DE)**

(54) **Air lift fermentor.**

(57) A reactor for the production of plasmids and bacteriophage uses a standard centrifuge bottle (10) to hold a sample. In the device, a column (16) is removably placed on the bottle (10) and has a gas delivery and venting system for aerating and mixing the sample. A port is disposed in the column for sampling and injection of reagents. At the completion of bacterial growth, the device is removed from the centrifuge bottle (10), which is transferred to a centrifuge for further processing.

FIG.1

EP 0 365 756 A1

## AIR SPARGE SYSTEM

### Field Of The Invention

This invention relates to a bioreactor and specifically an air lift fermentor used to produce plasmid and bacteriophage in bacterial cultures.

### Related Art

Devices which are used to incubate and produce living organisms such as bacteria for research activities have been used in laboratories and industrial applications. These devices are used in molecular biology research and in particular in the production of a bacteria for DNA cloning. DNA is one of the basic targets in molecular biology research. Classical methods of study have been impeded by limited amounts of DNA that could be obtained by extraction from an organism. Moreover, cloning of a specific gene will yield even less DNA. The ability of a plasmid or bacteriophage to reproduce within a host bacteria provides a technique of amplifying cloned DNA. In general, this process is accomplished by inserting a specific fragment of DNA into the plasmid or bacteriophage genome after which, the plasmid or bacteriophage it is put into a bacterial host. Once within the host, this vector replicates producing hundreds of identical DNA copies per bacterium.

At an appropriate time the vector is harvested and the DNA purified. Following isolation and purification of the vector, the specific insert DNA can be excised and further purified. Greater than a million fold increase in specific gene DNA can be realized by this technique.

For purposes of reference, the generally accepted protocol for the production of plasmid and bacteriophage DNA is described in "A Laboratory Guide to Molecular Cloning", T. Maniatis, et al. Essentially, the procedures for isolating plasmid and bacteriophage DNA are virtually the same. In both cases., the infected host is introduced into a flask containing a suitable liquid nutrient medium. The culture is shaken at 37°C. During the 9 to 18 hours incubation period, samples are taken to determine the extent of bacterial growth.

Within the art there are two techniques for expansion of bacterial cultures to achieve this purpose. For research applications, cultures are expanded in flasks housed in an incubator shaker. Final volumes range from 50 ml to 6 liters per unit. Large fermentation systems are in general used for large scale industrial applications. The incubator shaker system provides proper temperature and aeration for bacterial growth by shaking the flask at approximately 250 RPM in a heated chamber. There are, however, numerous shortcomings in this system. First, the incubator shaker is an expensive piece of equipment, costing between $4,500 and $6,000. Secondly, the shakers occupy roughly 8 square feet each of floor space and have a capacity of either six 2.8-liter or twenty 250 ml flasks. The large 2.8 liter flasks required to grow one liter cultures require large amounts of sterile hood space and are difficult to clean, autoclave and store. That is, five flasks take up four square feet.

In addition to these shortcomings, glass breakage poses safety problems and may result in sample loss. Monitoring of cell density requires that the flasks be removed from the shaker and sampling performed in the sterile hood. Sample loss or mix up may also occur when transferring the culture to centrifuge bottles at the completion of the growth phase. Given these deficiencies in size, cost, and use a second class of device is represented by the Kontes "glass airlift bioreactor" sold by Kontes Life Science Products of Vineland, New Jersey and has also been marketed. This device offers improvements in terms of the size and cost. However, handling problems still exist and the body itself is not readily transferable for purposes of centrifuging.

### Summary Of The Invention

Given the deficiencies of the prior art, it is an object of this invention to provide an apparatus for mixing and aerating bacteria by fitting it to standard centrifuge containers.

It is another object of this invention to provide an air sparge device which is low in cost, easy to use and efficient, which uses a standard laboratory water bath for temperature control rather than the expensive incubator-shaker.

Yet another object of this invention is to provide for an apparatus which allows the production of plasmid and bacteriophage in a reliable and safe manner.

These and other objects of this invention are accomplished by means of an apparatus which employs a standard 1-liter centrifuge bottle as the container for mixing and aerating bacteria. The apparatus is attached to the top of the centrifuge bottle and is equipped with a compatible fitting which has a filtered input for aeration, a vent, and a sample port. By providing for a technique of defoaming utilizing a wholly passive technique, the use of this system is not labor intensive and may proceed unimpeded without the need for constant and frequent inspections.

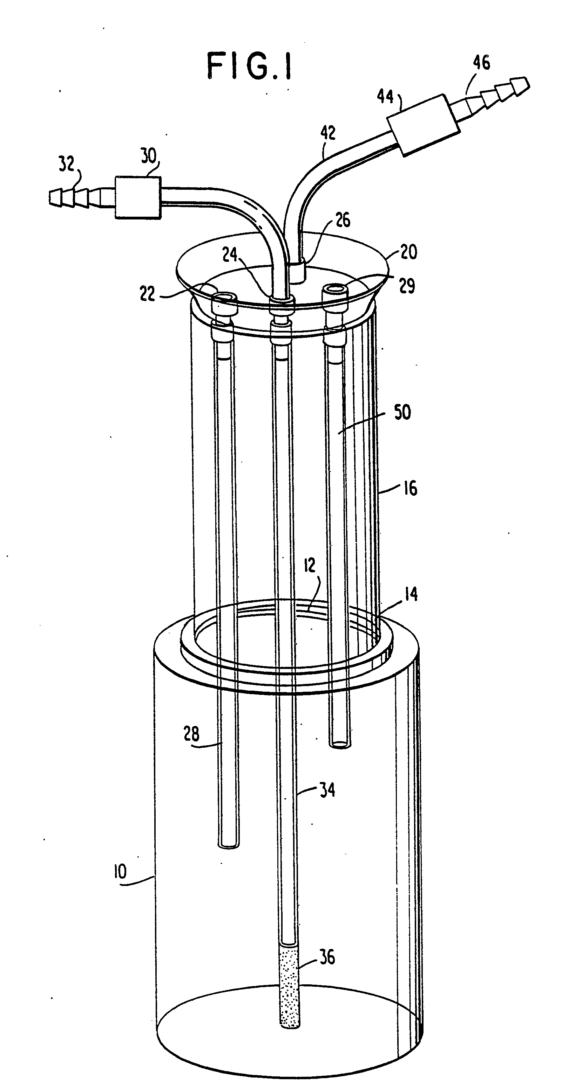

This invention will be described in greater detail by referring to the attached drawing and the description of the preferred embodiment that follows.

Brief Description Of The Drawing

The sole Figure in this case is a perspective view of the complete device in accordance with this invention.

A Description Of The Preferred Embodiment

Referring now to the sole Figure, the fermenting system of this invention utilizes a standard 1-liter centrifuge bottle 10 as the container in which the growth media and bacteria are housed. This centrifuge bottle is generally made of plastic but alternatively any Nalgene labware bottle or carboy may be used. The container 10 has a neck portion which is threaded 12 to receive a threaded flange portion 14. The threaded flange portion 14 screws on to the threads 12 such that when mixing and aeration is complete, flange 14 is removed and a standard cap is used to house the mixture for purposes of transfer to a centrifuge.

The flange 14 is fitted to a hollow cylinder 16. The cylinder 16 has an inside wall diameter which is approximately the same as the outside wall diameter of the fitting 14. The top end of the sleeve 16 receives cap 20 which is either threaded or of a snap-on configuration. Four connector pieces 22, 24, 26 and 29 provide couplings between exterior fittings and the interior of the chamber formed by the centrifuge bottle 10. Fitting 22 has coupled to its internal nipple a conduit 28. The conduit, made of a plastic material, defines with the fitting 22 a sample port for purposes of injection or extraction of materials to and from the reaction chamber in the container 10.

Coupled to fitting 24 via an external nipple is a plastic conduit 34 coupled at its opposite end to an air input filter 30. The filter 30 has a barbed fitting 32 which is coupled to a source of air, typically by air pump or the like. The internal nipple coupled to the fitting 24 has a plastic conduit 34 which terminates in an air stone 36. The air stone 36 is disposed at the bottom of the chamber 10. Alternately, nozzle 46 can be coupled to a vacuum source which will draw air into the air inlet port 24. By either technique oxygen is supplied for purposes of mixing and aeration.

Coupled to fitting 26 is a plastic conduit 42 which is coupled to vent 44 having housed therein carbon or other suitable filtering material. The nozzle section 46 may, if necessary, be placed in fluid communication with an exhaust system for purposes of positively venting gases from the chamber. Fitting 29 and plastic conduit 50 are provided as auxiliary fittings for special use by the user.

In operation the standard 1 liter centrifuge bottle 10 is loaded with nutrient media and the sample. The sleeve 16 is then mounted and a nipple 32 is either coupled to a source of air pressure, or nipple 46 is coupled to a vacuum source. The bottle 10 is incubated at 37° C in an incubator or by immersion in a standard laboratory water bath. Samples can be taken or reagents added to the culture through syringe or luer lock fitting 22 utilizing plastic conduit 28. During incubation, the researcher can grow and expand any volume of culture from a 100 ml initial sample to 10 liters per unit. Due to its compact size, twelve 1-liter cultures can be expanded utilizing a standard 1 foot x 1.5 foot water bath.

Moreover, 50 individual 1-liter cultures can be handled in the same space previously required utilizing an incubator shaker (8 square feet).

When the sample has been fully incubated, the sleeve 16 and fitting 14 are removed and the bottle 10 capped. Bottle 10 is then transported directly to a centrifuge for further processing.

As can be appreciated, utilizing this non-breakable, compact device permits safe and inexpensive growth of bacteria for the production of both plasmids and bacteriophages.

It is apparent that modifications of this device can be practiced without departing from the essential scope thereof.

Claims

1. A device for mixing and aerating a material comprising:
a container for receiving said material;
a hollow transition piece mounted to said container;
a column fitted to said transition piece; and
supply means for delivering gas into said container for aerating and mixing.

2. The device of Claim 1 further comprising vent means coupled to said column.

3. The device of Claim 1 further comprising a sampling port mounted on said column, said sampling port providing access to said container for extracting a sample of said material or introducing a reagent into said container.

4. The device of Claim 1 wherein said container is a one liter centrifuge bottle and said transition piece is removable therefrom to allow said bottle to be capped.

5. The device of Claim 4 wherein said bottle has a threaded opening and said transition piece screws onto said threaded opening, said transition piece having an upwardly extending collar from said opening and said column is integral to said collar.

6. The device of Claim 5 wherein said column has a top and said supply means is mounted to said top.

7. The device of Claim 6 wherein said supply means comprises a filter coupled to a syringe mounted to said top, a conduit coupled to said syringe and extending into said conduit and a nozzle mounted to said conduit for ejecting a stream of gas into said sample.

8. The device of Claim 6 further comprising vent means mounted to said top.

9. The device of Claim 6 further comprising a sampling port mounted to said top.

10. A device for reacting an input material by aeration comprising:
a centrifuge container receiving said material;
a transition piece mounted on an opening of said container;
a hollow column mountable on said transition piece; and
supply means mounted to said column to deliver a gas into said container for aerating said material.

11. The device of Claim 10 further comprising vent means coupled to said column.

12. The device of Claim 10 further comprising a sampling port mounted on said column, said sampling port providing access to said container for extracting a sample of said material or introducing a reagent into said container.

13. The device of Claim 10 wherein said container is a one liter centrifuge bottle and said transition piece is removable therefrom to allow said bottle to be capped.

14. The device of Claim 13 wherein said bottle has a treaded opening and said transition piece screws onto said threaded opening.

15. The device of Claim 14 wherein said column has a top, and said supply means is mounted to said top.

16. The device of Claim 15 wherein said supply means comprises a filter coupled to a syringe mounted to said top, a conduit coupled to said syringe and extending into said conduit and a nozzle mounted to said conduit for ejecting a stream of gas into said sample.

17. The device of Claim 15 further comprising vent means mounted to said top.

18. The device of Claim 15 further comprising a sampling port mounted to said top.

# FIG.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CH-A- 380 683 (MILES LAB. INC.)<br>* Page 1, line 55 - page 2, line 5; figure 1 * | 1-3 | C 12 M 1/24<br>B 01 L 3/00<br>C 12 M 1/04 |
| A | | 4,7-13, 16-18 | |
| A | CH-A- 582 541 (INFORS AG)<br>* Column 1, line 35 - column 2, line 14; claim 1, subclaims 1,2,5; figure 1 * | 1,2,6-8 ,10,11, 15-17 | |
| A | US-A-3 701 434 (H.C. MOORE)<br>* Column 3, line 36 - column 4, line 46; column 4, line 63 - column 5, line 2; claim 1; figure 1 * | 1,2,4,5 ,10,13, 14 | |

TECHNICAL FIELDS
SEARCHED (Int. Cl.5)

C 12 M
B 01 L

The present search report has been drawn up for all claims

| Place of search | Date of completion of the search | Examiner |
|---|---|---|
| THE HAGUE | 05-12-1989 | GROENENDIJK M.S.M. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)